(19) Europäisches Patentamt
European Patent Office
Office européen des brevets

(11) Veröffentlichungsnummer: **0 036 153 B1**

(12) # EUROPÄISCHE PATENTSCHRIFT

(45) Veröffentlichungstag der Patentschrift: **02.11.83**

(51) Int. Cl.³: **C 07 D 249/08**, A 61 K 31/41

(21) Anmeldenummer: **81101668.2**

(22) Anmeldetag: **07.03.81**

(54) **1,1-Diphenyl-2-(1,2,4-triazol-1-yl)-äthan-1-ole als Antimykotika.**

(30) Priorität: **15.03.80 DE 3010093**

(43) Veröffentlichungstag der Anmeldung: **23.09.81 Patentblatt 81/38**

(45) Bekanntmachung des Hinweises auf die Patenterteilung: **02.11.83 Patentblatt 83/44**

(84) Benannte Vertragsstaaten: **AT BE CH DE FR GB IT LI LU NL SE**

(56) Entgegenhaltungen:
**DE - A - 2 324 010**
**DE - A - 2 431 407**
**DE - A - 2 547 954**
**DE - A - 2 654 890**
**DE - A - 2 737 489**

(73) Patentinhaber: **BASF Aktiengesellschaft, Carl-Bosch-Strasse 38, D-6700 Ludwigshafen (DE)**

(72) Erfinder: **Scharwaechter, Peter, Dr., An der Duene 9, D-2082 Moorrege (DE)**
Erfinder: **Gutsche, Klaus, Dr., Ehmschenkamp 5, D-2084 Rellingen (DE)**
Erfinder: **Heberle, Wolfgang, Dr., An der Duene 4, D-2082 Moorrege (DE)**
Erfinder: **Wesenberg, Walter, Dr., Dorfstrasse 16, D-2421 Bujendorf über Eutin (DE)**
Erfinder: **Kohlmann, Friedrich-Wilhelm, Dr., An der Duene 6, D-2082 Moorrege (DE)**

EP 0 036 153 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 1,1-Diphenyl-2-(1,2,4-triazol-1-yl)-äthan-1-ole als Antimykotika

Die Erfindung betrifft 1,1-Diphenyl-2-(1,2,4-triazol-1-yl)-äthan-1-ole und ihre pharmakologisch verträglichen Säureadditionssalze als Antimykotika zur innerlichen oder äusserlichen Anwendung.

Es sind zahlreiche antimykotische Mittel, z.B. Miconazol oder Clotrimazol, bekannt. Ihre Wirkungen befriedigen nicht immer. Dieser Erfindung liegt die Aufgabe zugrunde, neue und wirksamere Antimykotika zur Verfügung zu stellen.

Es wurde nun gefunden, dass Verbindungen der allgemeinen Formel I

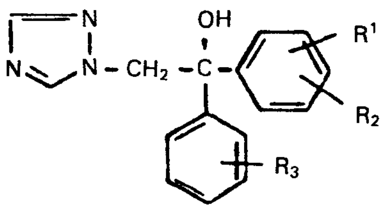

(I)

in der einer der Reste $R^1$ bis $R^3$ ein Chloratom bedeutet und die anderen, die gleich oder verschieden voneinander sein können, ein Wasserstoffatom oder Chloratom bedeuten, sowie deren pharmakologisch verträgliche Säureadditionssalze wertvolle pharmakologische Eigenschaften aufweisen.

Verbindungen der allgemeinen Formel I fallen zwar unter den Anspruch 1 der DE-A-2 654 890, sind dort aber nicht konkret genannt. Sie sind jedoch in der nachveröffentlichten älteren europäischen Anmeldung 15 756 als Fungizid zur Anwendung im Pflanzenschutz beschrieben. Eine Wirksamkeit als Antimykotika zur Behandlung von Mensch und Tier ist in beiden Schriften weder erwähnt noch nahegelegt. Von den erfindungsgemässen Verbindungen der allgemeinen Formel I sind diejenigen Verbindungen, in denen der Substituent $R^1$ in para-Stellung zur Äthylkette steht, und die der allgemeinen Formel II entsprechen,

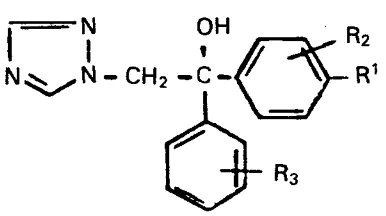

. (II)

sowie deren pharmakologisch verträgliche Säureadditionsprodukte besonders bevorzugt.

Als übliche Säuren zur Bildung pharmakologisch verträglicher Salze kommen insbesondere Salpetersäure, Salzsäure, Schwefelsäure, Phosphorsäure, Essigsäure, Proprionsäure, Milchsäure, Bernsteinsäure, Weinsäure, Citronensäure, Benzoesäure, Salicylsäure, und Nicotinsäure in Frage oder können beispielsweise Fortschritte der Arzneimittelforschung, Band 10, Seite 224 bis 225, Birkhäuser Verlag, Basel und Stuttgart, 1966, entnommen werden.

Bevorzugt werden die genannten anorganischen Säuren, insbesondere Salpetersäure oder Salzsäure, die mit den erfindungsgemässen Verbindungen besonders gut kristallisierende Salze bilden.

Die erfindungsgemässen Antimykotika, bei denen die Reste $R^1$ bis $R^3$ voneinander verschieden sind oder bei denen die Phenylreste einen gleichen Substituenten in unterschiedlicher Stellung tragen, weisen mit dem C-Atom, das die Hydroxylgruppe trägt, ein Asymmetriezentrum auf. Die Trennung, Isolierung oder Herstellung eines bestimmten optischen Isomeren kann in an sich üblicher Weise erfolgen. Die optischen Isomeren und ihre Herstellung sollen von der Erfindung mitumfasst werden.

Die Verbindungen der allgemeinen Formel I und ihre Säureadditionssalze werden hergestellt, indem man eine Verbindung der allgemeinen Formel III

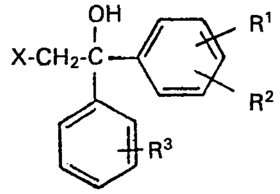

(III)

in der $R^1$ bis $R^3$ die für die allgemeine Formel I angegebenen Bedeutungen haben und X für ein Halogenatom, vorzugsweise ein Chlor- oder Bromatom steht, mit 1,2,4-Triazol der Formel IV

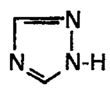

(IV)

in an sich üblicher Weise umsetzt und gegebenenfalls die so erhaltene Verbindung in ein pharmakologisch verträgliches Säureadditionssalz überführt.

Diese Reaktion wird zweckmässigerweise mit stöchiometrischen Mengen in Gegenwart eines säurebindenden Mittels oder mit überschüssigem 1,2,4-Triazol, sowohl in Gegenwart als auch in Abwesenheit eines Lösungs- oder Verdünnungsmittels durchgeführt.

Als Verdünnungsmittel werden vorwiegend organische Lösungsmittel, beispielsweise Dimethylformamid, Hexamethylphosphortriamid, Acetonitril oder Benzol verwendet.

Als säurebindendes Mittel eignet sich vorzugsweise ein Überschuss an 1,2,4-Triazol oder eines der üblichen Säurebindungsmittel, wie z.B. Hydroxide, Carbonate und Alkoholate von Alkali- und Erdalkalimetallen, insbesondere des Natriums, Kaliums, Magnesiums oder Calciums, sowie organische Basen, wie beispielsweise Pyridin oder tertiäre Amine, wie Triäthylamin.

Die Reaktionstemperaturen können in einem grösseren Bereich variiert werden. Im allgemeinen arbeitet man bei Temperaturen von 0 bis 150°C, vorzugsweise von 30 bis 120°C.

Die Herstellung der Ausgangsverbindungen der allgemeinen Formel III kann in an sich bekannter Weise nach Methoden zur Darstellung tertiärer Alkohole aus Ketonen oder Carbonsäurederivaten und Grignard-Verbindungen durchgeführt werden, wie es beispielsweise in Houben-Weyl, Methoden der organischen Chemie, Band *13/2a,* 46-527 (1973) beschrieben wird.

Verwendet man beispielsweise 1-(2,4-Dichlorphenyl)-1-(4-chlorphenyl)-2-chlor-äthan-1-ol, dargestellt aus 2,2',4'-Trichloracetophenon und 4-Chlorphenyl-magnesiumbromid und 1,2,4-Triazol als Ausgangsprodukte, so kann der Reaktionsverlauf durch folgendes Formelschema wiedergegeben werden:

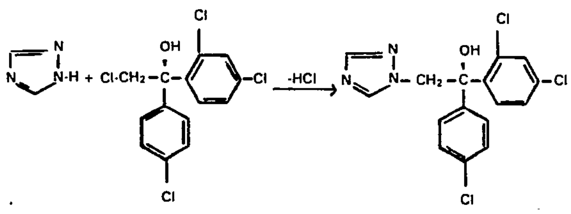


Die Verbindungen der allgemeinen Formel I und ihre Salze besitzen ausgezeichnete chemotherapeutische, insbesondere antimykotische Eigenschaften.

Gegenstand der Erfindung ist daher auch ein antimykotisches Mittel, gekennzeichnet, durch einen Gehalt an einer Verbindung der allgemeinen Formel I oder deren pharmakologisch verträgliches Säureadditionssalz als Wirkstoff neben üblichen Träger- und Verdünnungsmitteln.

Die überraschend gute antimykotische Wirksamkeit in vitro wurde in folgendem Test gegen Candida albicans aufgezeigt:

Pseudomycel- und Mycelphasentest mit Candida albicans:

Dieser Test berücksichtigt, dass C. albicans in vivo nicht nur Sprosszellen, sondern auch Pseudomycel und echtes Mycel ausbilden kann (Dimorphie). In vitro wird die Ausbildung aller Wachstumsphasen dieses Keimes und der Einfluss von antimycetisch wirksamen Substanzen in folgender Weise geprüft: Candida albicans (Robin) Berkhout No 20/M (Stamm der Mykothek, Nordmark-Werke GmbH) wird mit der Impföse einerseits auf die Oberfläche A des Nährmediums (Brain Heart Infusion, Difco) und andererseits bei B als Stichkultur angeimpft (Figur).

Im Bereich der Stichkultur wird zusätzlich ein Deckglas C aufgelegt (mikroaerophile Bedingungen). Die Petrischale D enthält im Nährmedium eine geometrisch abgestufte Verdünnungsreihe der jeweiligen Prüfsubstanz. Nach Inkubation dieser Testplatten (2 Tage, 37 °C) sind in präparatefreien Kontrollplatten im aeroben Bezirk A ausschliesslich Sprosszellen (Hefephase), im mikroaerophilen Bezirk B ausschliesslich Pseudohyphen und echte Hyphen gewachsen.

Als minimale Hemmkonzentrationen wurden diejenigen Konzentrationsstufen des Prüfpräparates bezeichnet, bei denen eine 100%ige Hemmung des jeweiligen Pilzwachstums zu beobachten war.

Diese Testanordnung ermöglicht es, zwischen der minimalen Hemmkonzentration der Hefephase ($MHK_{Hefephase}$) und der MHK der Pseudomycelphase bzw. Mycelphase ($MHK_{Mycelphase}$) für Candida albicans zu unterscheiden.

Während die MHK-Werte für die Hefephase bei über 128 $\mu$g/ml liegen, ergeben sich für die Mycelphase äusserst niedrige MHK-Werte, wie aus der Tabelle 1 hervorgeht.

Tabelle 1

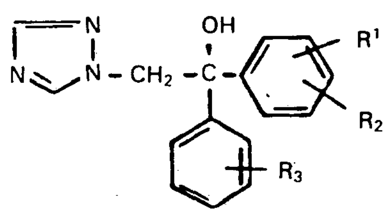 , (I)

| Beispiel Nr. | $R^1$ | $R^2$ | $R^3$ | Salz | $MHK_{Mycelph.}$ ($\mu$g/ml) | $LD_{50}$ (Maus) p.o. (mg/kg) |
|---|---|---|---|---|---|---|
| 1 | H | 4-Cl | 4-Cl | – | <0,125 | >1000 |
| 2 | H | 4-Cl | 4-Cl | HCl | <0,125 | >1000 |
| 3 | 2-Cl | 4-Cl | 4-Cl | – | <0,125 | >4000 |
| 4 | 2-Cl | 4-Cl | 4-Cl | HCl | <0,125 | >1000 |
| 5 | 2-Cl | 4-Cl | 4-Cl | $HNO_3$ | <0,125 | >1000 |
| 6 | 2-Cl | H | H | HCl | <0,125 | >1000 |
| 7 | H | 4-Cl | H | HCl | <0,125 | >1000 |
| 8 | H | 4-Cl | H | $HNO_3$ | <0,125 | >1000 |
| 9 | 2-Cl | 4-Cl | H | – | <0,125 | >1000 |
| 10 | 2-Cl | 4-Cl | H | HCl | <0,125 | >1000 |
| 11 | 2-Cl | 4-Cl | H | $HNO_3$ | <0,125 | >1000 |
| 12 | 2-Cl | H | 4-Cl | HCl | 1 | >1000 |
| 13 | 2-Cl | 4-Cl | 2-Cl | – | 1 | >1000 |
| 14 | 2-Cl | 4-Cl | 2-Cl | HCl | 1 | >1000 |
| 15 | 2-Cl | 4-Cl | 3-Cl | – | <0,125 | >1000 |
| 16 | 2-Cl | 4-Cl | 3-Cl | HCl | 1 | >1000 |

Die antimikrobielle Wirksamkeit in vitro gegen Dermatophyten wurde im Agar-Dilutionstest (Lit.: P. Klein «Bakteriologische Grundlagen der chemotherapeutischen Laboratoriumspraxis») ermittelt. Das Ergebnis dieser Untersuchungen am Beispiel der Verbindung Nr. 3 zeigt Tabelle 2.

Tabelle 2

| *Mikrosporon-Arten:* | | MHK (µg/ml) |
|---|---|---|
| M. audouinii | No. 4 | 0,25 |
| M. ferrugineum | No. 5 | 0,5 |
| M. canis | No. 167 | 4 |
| M. canis | No. 168 | 2 |
| M. canis | No. 169 | 8 |
| *Trichophyton-Arten:* | | |
| T. schoenleinii | No. 9 | 0,25 |
| T. violaceum | No. 10 | 2 |
| T. mentagroph. | No. 159 | 0,125 |
| T. mentagroph. | No. 172 | 0,25 |
| T. mentagroph. | No. 173 | 0,25 |
| *Epidermophyten-Arten:* | | |
| E. floccosum | No. 157 | 16 |

Experimentelle Candidose der Maus durch Candida albicans.

Zur Feststellung der oralen Wirksamkeit wurden Gruppen von jeweils 10 ca. 20 g schweren Mäusen 2 Tage mit je 50 mg/kg Hydrocortison i.m. vorbehandelt, um ein gutes Angehen der Infektion zu erzielen.

Die Mäuse wurden dann mit je 500.000 Candida albicans-Keimen i.v. infiziert und danach 4 Tage lang zweimal täglich mit je 100 mg/kg der zur prüfenden Substanz oral behandelt.

Neben der infizierten Gruppe und der nicht behandelten Kontrollgruppe wurde zum Vergleich je eine Gruppe mit den Referenzsubstanzen Miconazol und Clotrimazol behandelt.

Aus der Tabelle 3 geht hervor, dass am 10. Tag post infectionem noch bis zu 100% der mit den erfindungsgemässen Antimykotika behandelten Tiere leben, während in der Kontrollgruppe und bei den mit den Referenzsubstanzen behandelten Tieren nur noch bis zu 30% überleben.

Tabelle 3 (Dosierung: 2 × tgl. 100 mg/kg)

| Beipiel | Anzahl der überlebenden Tiere am . . . . Tag post infectionem | | | | | | | | | |
|---|---|---|---|---|---|---|---|---|---|---|
| Nr. | 1. | 2. | 3. | 4. | 5. | 6. | 7. | 8. | 9. | 10. |
| 1 | 10 | 10 | 10 | 10 | 10 | 10 | 10 | 10 | 10 | 9 |
| 2 | 10 | 10 | 10 | 10 | 10 | 10 | 9 | 9 | 9 | 9 |
| 3 | 10 | 10 | 10 | 10 | 10 | 10 | 10 | 10 | 10 | 10 |
| 4 | 10 | 10 | 10 | 10 | 10 | 10 | 10 | 10 | 10 | 10 |
| 5 | 10 | 10 | 10 | 10 | 10 | 10 | 10 | 10 | 10 | 10 |
| 6 | 10 | 10 | 10 | 10 | 10 | 10 | 10 | 10 | 9 | 9 |
| 7 | 10 | 10 | 10 | 10 | 10 | 10 | 10 | 10 | 10 | 10 |
| 8 | 10 | 10 | 10 | 10 | 10 | 10 | 10 | 10 | 10 | 10 |
| 9 | 10 | 10 | 10 | 10 | 10 | 10 | 10 | 10 | 10 | 10 |
| 10 | 10 | 10 | 10 | 10 | 10 | 10 | 10 | 10 | 10 | 10 |
| 11 | 10 | 10 | 10 | 10 | 10 | 10 | 10 | 9 | 9 | 9 |
| 15 | 10 | 10 | 10 | 10 | 10 | 10 | 9 | 9 | 8 | 8 |
| Miconazol | 10 | 10 | 8 | 8 | 6 | 4 | 2 | 2 | 1 | 1 |
| Clotrimazol | 10 | 10 | 10 | 9 | 8 | 6 | 5 | 5 | 4 | 3 |
| Kontrolle | 10 | 10 | 8 | 7 | 4 | 3 | 2 | 1 | 1 | 1 |

Zur Prüfung der Wirkungsintensität in vivo wurden Gruppen von jeweils 10 Mäusen, wie oben beschrieben, mit Candida albicans-Keimen infiziert und anschliessend 4 Tage lang zweimal täglich mit 46,4 mg/kg (Tabelle 4) bzw. 21,5 mg/kg (Tabelle 5) einer Auswahl von Prüfsubstanzen behandelt.

Tabelle 4 (Dosierung: 2 × tgl. 46,4 mg/kg)

| Beipiel | Anzahl der überlebenden Tiere am . . . . Tag post infectionem | | | | | | | | | |
|---|---|---|---|---|---|---|---|---|---|---|
| Nr. | 1. | 2. | 3. | 4. | 5. | 6. | 7. | 8. | 9. | 10. |
| 2 | 10 | 10 | 10 | 10 | 10 | 10 | 10 | 10 | 9 | 9 |
| 3 | 10 | 10 | 10 | 10 | 10 | 10 | 10 | 10 | 10 | 10 |
| 4 | 10 | 10 | 10 | 10 | 10 | 10 | 10 | 10 | 9 | 9 |
| 5 | 10 | 10 | 10 | 10 | 10 | 10 | 10 | 9 | 9 | 9 |
| 11 | 10 | 10 | 10 | 10 | 10 | 10 | 10 | 10 | 9 | 9 |
| Clotrimazol | 10 | 10 | 9 | 8 | 8 | 7 | 6 | 6 | 4 | 3 |
| Kontrolle | 10 | 10 | 8 | 7 | 4 | 3 | 1 | 1 | 1 | 1 |

Tabelle 5

(Dosierung: 2 × tgl. 21,5 mg/kg)

| Beipiel Nr. | Anzahl der überlebenden Tiere am . . . . Tag post infectionem 1. | 2. | 3. | 4. | 5. | 6. | 7. | 8. | 9. | 10. |
|---|---|---|---|---|---|---|---|---|---|---|
| 2 | 10 | 10 | 10 | 10 | 10 | 9 | 9 | 8 | 8 | 8 |
| 3 | 10 | 10 | 10 | 10 | 10 | 10 | 10 | 10 | 10 | 10 |
| 4 | 10 | 10 | 10 | 10 | 10 | 10 | 10 | 10 | 10 | 10 |
| 5 | 10 | 10 | 10 | 10 | 10 | 10 | 10 | 10 | 10 | 10 |
| 11 | 10 | 10 | 10 | 10 | 10 | 10 | 10 | 9 | 9 | 9 |
| Clotrimazol | 10 | 10 | 9 | 5 | 5 | 3 | 2 | 2 | 0 | 0 |
| Kontrolle | 10 | 10 | 8 | 5 | 3 | 1 | 1 | 1 | 0 | 0 |

Erläuterungen zu den Tabellen 3, 4 und 5:
Beispiel-Nr.

1 = 1,1-Bis-(4-chlorphenyl)-2-(1,2,4-triazol-1-yl)-äthan-1-ol
2 = 1,1-Bis-(4-chlorphenyl)-2-(1,2,4-triazol-1-yl)-äthan-1-ol-hydrochlorid
3 = 1-(4-Chlorphenyl)-1-(2,4-dichlorphenyl)-2-(1,2,4-triazol-1-yl)-äthan-1-ol
4 = 1-(4-Chlorphenyl)-1-(2,4-dichlorphenyl)-2-(1,2,4-triazol-1-yl)-äthan-1-ol-hydrochlorid
5 = 1-(4-Chlorphenyl)-1-(2,4-dichlorphenyl)-2-(1,2,4-triazol-1-yl)-äthan-1-ol-nitrat
6 = 1-Phenyl-1-(2-chlorphenyl)-2-(1,2,4-triazol-1-yl)-äthan-1-ol-hydrochlorid
7 = 1-Phenyl-1-(4-chlorphenyl)-2-(1,2,4-triazol-1-yl)-äthan-1-ol-hydrochlorid
8 = 1-Phenyl-1-(4-chlorphenyl)-2-(1,2,4-triazol-1-yl)-äthan-1-ol-nitrat
9 = 1-Phenyl-1-(2,4-chlorphenyl)--2-(1,2,4-triazol-1-yl)-äthan-1-ol
10 = 1-Phenyl-1-(2,4-dichlorphenyl)-2-(1,2,4-triazol-1-yl)-äthan-1-ol-hydrochlorid
11 = 1-Phenyl-1-(2,4-dichlorphenyl)-2-(1,2,4-triazol-1-yl)-äthan-1-ol-nitrat
15 = 1-(3-Chlorphenyl)-1-(2,4-dichlorphenyl)-2-(1,2,4-triazol-1-yl)-äthan-1-ol.

Die Ergebnisse zeigen, dass die erfindungsgemässen Antimykotika der allgemeinen Formel I in vivo den in der medizinischen Praxis eingeführten Antimykotika Miconazol {1-[2,4-Dichlor-β-(2,4-dichlorbenzyloxy)-phenäthyl]-imidazolnitrat} und Clotrimazol [1-(o-Chlor-α,α-diphenyl-benzyl)-imidazol], beispielsweise an der mit Candida albicans infizierten Maus, deutlich überlegen sind.

Da die bislang verfügbaren Heilmittel zur Behandlung von Systemmykosen nicht befriedigen und mit einer weiteren Zunahme dieser Erkrankungen gerechnet wird [Lit.: Infection *2*, 95 (1974); Chemotherapy *22*, 1 (1976); Dtsch. Apoth. Ztg. *118*, 1269 (1978)], stellen die erfindungsgemässen Antimykotika eine echte Bereicherung des Arzneimittelschatzes dar.

Die erfindungsgemässen Antimykotika sind besonders geeignet zur oralen und äusserlichen Behandlung von Pilzinfektionen an Mensch und Tier.

Als Indikationsgebiete an Mensch und Tier sind beispielsweise: Dermatomykosen, Dermatophytosen und Systemmykosen, insbesondere verursacht durch Dermatophyten, wie Species der Gattungen Epidermophyten, Microsporum oder Trichophyton, Hefen, wie Species der Gattung Candida, und Schimmelpilze, wie Species der Gattungen Aspergillus, Mucor oder Absidia zu nennen.

Die Antimykotika können allein oder zusammen mit anderen bekannten Wirkstoffen, insbesondere Antibiotika, verwendet werden.

Die Herstellung der chemotherapeutischen Mittel oder Zubereitungen, die mit üblichen festen, halbfesten oder flüssigen Trägerstoffen oder Verdünnugsmitteln und den üblicherweise verwendeten pharmazeutisch-technischen Hilfsstoffen entsprechend der gewünschten Applikationsart mit einer zur innerlichen oder äusserlichen Anwendung geeigneten Dosierung erfolgt in üblicher Weise, insbesondere durch Vermischen (vgl. L.G. Godman, A. Gilman, The Pharmacological Basis of Therapeutics).

Als innerliche und äusserliche Darreichungsformen kommen beispielsweise Tabletten, Dragees, Kapseln, Pillen, wässrige Lösungen, Suspensionen und Emulsionen, gegebenenfalls sterile injizierbare Lösungen, nichtwässrige Emulsionen, Suspensionen und Lösungen, Salben, Cremes, Pasten, Lotions, Puder, Gele oder Sprays in Betracht.

Im allgemeinen hat es sich sowohl in der Humanals auch in der Veterinärmedizin als vorteilhaft erwiesen, den oder die Wirkstoffe in Mengen von etwa 10 bis etwa 250 mg/kg Körpergewicht je 24 Stunden, vorzugsweise in Form mehrerer Einzelgaben zur Erzielung der gewünschten Ergebnisse zu verabreichen. Es kann jedoch erforderlich sein, von den genannten Dosierungen abzuweichen, und zwar in Abhängigkeit von der Art und der Schwere der Erkrankung, der Art der Zubereitung und der Applikation des Arzneimittels, sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

Bei äusserlicher, lokaler Anwendung enthalten die Zubereitung 0,5 bis 5, bevorzugt 1 bis 2 Gew.-% Wirkstoff. Bei oraler Gabe kommen als Einzeldosierungen Mengen von 50 bis 250 mg in Betracht. In der Regel wird der Wirkstoff täglich 1 bis 4 mal verabreicht.

Die üblichen galenischen Applikationsformen, wie Tabletten, können beispielsweise durch Mischen des Wirkstoffs mit bekannten Hilfsstoffen, beispielsweise inerten Verdünnungsmitteln, wie Dextrose, Zukker, Sorbit, Mannit, Polyvinylpyrrolidon, Calciumcarbonat, Calciumphosphat oder Milchzucker, Sprengmitteln, wie Maisstärke oder Alginsäure, Bindemitteln, wie Stärke oder Gelatine, Gleitmitteln, wie Magnesiumstearat oder Talk, und/oder Mitteln zur Erzielung des Depoteffektes, wie Carboxypolymethylen, Carboxymethylcellulose, Celluloseacetatphthalat oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspension mit den erfindungsgemäss zu verwendenden Wirkstoffen können zusätzlich geschmacksverbessernde Mittel, wie Saccharin, Cyclamat oder Zucker sowie z.B. Aromastoffe, wie Vanillin oder Orangenextrakt, enthalten. Sie können ausserdem Suspendierhilfsstoffe, wie Natriumcarboxymethylcellulose, oder Schutzstoffe, wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Salben, Pasten, Cremes und Gele für die äusserliche Anwendung können neben dem oder den Wirkstoffen die üblichen Trägerstoffe enthalten, z.B. tierische und pflanzliche Fette, Wachse, Paraffine, Stärken, Tragant, Cellulosederivate, Polyäthylenglykole, Silicone, Bentonite, Kieselsäure, Talkum und Zinkoxid oder Gemische dieser Stoffe.

Puder und Sprays können neben dem Wirkstoff die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe, wie Lösungsmittel, Lösungsvermittler und Emulgatoren, z.B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnussöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

*Beispiel 1*

58,6 g 1-Brom-4-chlorbenzol werden in 250 ml Diäthyläther mit 8 g Magnesiumspänen bei Siedetemperatur zur Reaktion gebracht und anschliessend bei 0 bis 10°C tropfenweisen mit 12,4 g Chloressigsäureäthylester versetzt. Nach einstündigem Rühren bei Raumtemperatur wird das Reaktionsgemisch mit Eis und wässriger Ammoniumchloridlösung behandelt. Die organische Phase wird über Natriumsulfat getrocknet und im Vakuum eingeengt. Das ölige Reaktionsprodukt wird nunmehr mit 20,8 g 1,2,4-Triazol und 8,4 g Natriumhydrogencarbonat 2 Stunden lang in der Schmelze bei 130°C Badtemperatur gerührt und anschliessend nach Abkühlung mit Wasser und Chloroform ausgeschüttelt. Die organische Phase wird mit Natriumsulfat getrocknet, eingeengt und der Rückstand mit Diäthyläther verrieben. Nach Umkristallisation aus 1,2-Dichloräthan erhält man 17,3 g kristallines 1,1-Bis-(4-chlorphenyl)-2-(1,2,4-triazol-1-yl)-äthan-1-ol mit dem Fp. 144°C.

$C_{16}H_{13}Cl_2N_3O$ (334,22)

| | | | | |
|---|---|---|---|---|
| ber.: | C 57,50 | H 3,92 | Cl 21,22 | N 12,57 |
| gef.: | C 57,4 | H 3,9 | Cl 21,4 | N 12,7 |

*Beispiel 2*

Durch Behandlung der Base aus Beispiel 1 mit Chlorwasserstoff, gelöst in Diisopropyläther, erhält man 1,1-Bis-(4-chlorphenyl)-2-(1,2,4-triazol-1-yl)-äthan-1-ol-hydrochlorid mit dem Fp. 117°C.

*Beispiel 3*

29,3 g 1-Brom-4-chlorbenzol werden in 250 ml Diäthyläther mit 4 g Magnesiumspänen bei Siedetemperatur zur Reaktion gebracht und anschliessend tropfenweise mit 22,4 g 2,2',4'-Trichloracetophenon versetzt. Nach einer Stunde wird mit wässriger Ammoniumchloridlösung zersetzt, die organische Phase neutral gewaschen, über Natriumsulfat getrocknet und im Vakuum eingeengt. Das ölige Reaktionsprodukt wird mit 20,8 g 1,2,4-Triazol und 3,4 g Natriumhydrogencarbonat in der Schmelze 2 Stunden bei 130°C Badtemperatur gerührt und nach Abkühlen mit Eiswasser und Chloroform ausgeschüttelt. Die organische Phase wird über Natriumsulfat getrocknet, eingeengt und mit Diäthyläther verrieben. Es werden 22,8 g kristallines 1-(4-Chlorphenyl)-1-(2,4-dichlorphenyl)-2-(1,2,4-triazol-1-yl)-äthan-1-ol mit dem Fp. 187°C erhalten.

$C_{16}H_{12}Cl_3N_3O$ (368,67)

| | | | | |
|---|---|---|---|---|
| ber.: | C 52,13 | H 3,28 | Cl 28,85 | N 11,40 |
| gef.: | C 52,06 | H 3,45 | Cl 29,4 | N 11,27 |

*Beispiel 4*

Durch Behandlung der Base aus Beispiel 3 mit Chlorwasserstoff, gelöst in Diisopropyläther, erhält man 1-(4-Chlorphenyl)-1-(2,4-dichlorphenyl)-2-(1,2,4-triazol-1-yl)-äthan-1-ol-hydrochlorid mit dem Fp. 130°C.

*Beispiel 5*

Durch Behandlung der Base aus Beispiel 3 mit 100%iger Salpetersäure, gelöst in Diisopropyläther, erhält man 1-(4-Chlorphenyl)-1-(2,4-dichlorphenyl)-2-(1,2,4-triazol-1-yl)-äthan-1-ol-nitrat mit dem Fp. 176°C.

*Beispiele 6 bis 16*

Analog zu den Beispielen 3 bis 5 wurden folgende Verbindungen synthetisiert:

6. 1-Phenyl-1-(2-chlorphenyl)-2-(1,2,4-triazol-1-yl)-äthan-1-ol-hydrochlorid Fp. 134°C.
7. 1-Phenyl-1-(4-chlorphenyl)-2-(1,2,4-triazol-1-yl)-äthan-1-ol-hydrochlorid Fp. 208°C.
8. 1-Phenyl-1-(4-chlorphenyl)-2-(1,2,4-triazol-1-yl)-äthan-1-ol-nitrat Fp. 201°C.
9. 1-Phenyl-1-(2,4-dichlorphenyl)-2-(1,2,4-triazol-1-yl)-äthan-1-ol Fp. 203°C.
10. 1-Phenyl-1-(2,4-dichlorphenyl)-2-(1,2,4-triazol-1-yl)-äthan-1-ol-hydrochlorid Fp. 183°C.
11. 1-Phenyl-1-(2,4-dichlorphenyl)-2-(1,2,4-triazol-1-yl)-äthan-1-ol-nitrat Fp. 214°C.
12. 1-(2-Chlorphenyl)-1-(4-chlorphenyl)-2-(1,2,4-triazol-1-yl)-äthan-1-ol-hydrochlorid Fp. 228°C.
13. 1-(2-Chlorphenyl)-1-(2,4-dichlorphenyl)-2-(1,2,4-triazol-1-yl)-äthan-1-ol Fp. 161°C.
14. 1-(2-Chlorphenyl)-1-(2,4-dichlorphenyl)-2-(1,2,4-triazol-1-yl)-äthan-1-ol-hydrochlorid Fp. 195°C.
15. 1-(3-Chlorphenyl)-1-(2,4-dichlorphenyl)-2-(1,2,4-triazol-1-yl)-äthan-1-ol Fp. 149°C.
16. 1-(3-Chlorphenyl)-1-(2,4-dichlorphenyl)-2-(1,2,4-triazol-1-yl)-äthan-1-ol-hydrochlorid Fp. 135°C.

*Beispiel 17*

Tablette mit 250 mg Wirkstoff

| | | | |
|---|---|---|---|
| Wirkstoff des Beispiels Nr. 3 1-(4-Chlorphenyl)-1-(2,4-dichlorphenyl)-2-(1,2,4-triazol-1-yl)-äthan-1-ol | | | 250 g |
| Kartoffelstärke | | | 100 g |
| Milchzucker | | | 50 g |
| Gelatinelösung | 4% | ca. | 45 g |
| Talkum | | | 10 g |
| 1000 Tabl. | = | ca. | 410 g |

Herstellung:

Der fein gepulverte Wirkstoff, Kartoffelstärke und Milchzucker werden gemischt. Die Mischung wird mit ca. 45 g 4% Gelatinelösung durchfeuchtet, feinkörnig granuliert und getrocknet. Das trockene Granulat wird gesiebt, mit 10 g Talkum vermischt und auf einer Rundläufer-Tablettiermaschine zu Tabletten verpresst. Die Tabletten werden in dicht schliessende Behälter aus Polypropylen gefüllt.

*Beispiel 18*

Creme mit 2% Wirkstoff

| | |
|---|---|
| Wirkstoff des Beispiels Nr. 3 1-(4-Chlorphenyl)-1-(2,4-dichlorphenyl)-2-(1,2,4-triazol-1-yl)-äthan-1-ol | 2,0 g |
| Glycerinmonostearat | 10,0 g |
| Cetylalkohol | 4,0 g |
| Polyäthylenglykol-400-stearat | 10,0 g |
| Polyäthylenglykol-sorbitanmonostearat | 10,0 g |
| Propylenglykol | 6,0 g |
| p-Hydroxybenzoesäuremethylester | 0,2 g |
| Demineralisiertes Wasser | ad 100,0 g |

Herstellung:

Der feinst gepulverte Wirkstoff wird mit Propylenglykol suspendiert und die Suspension in die auf 65°C erwärmte Schmelze aus Glycerinmonostearat, Cetylalkohol, Polyäthylenglykol-400-stearat und Polyäthylenglykolsorbitanmonostearat gerührt. In diese Mischung wird die 70°C heisse Lösung des p-Hydroxybenzoesäuremethylesters in Wasser emulgiert. Nach dem Erkalten wird die Creme über eine Kolloidmühle homogenisiert und in Tuben abgefüllt.

*Beispiel 19*

Puder mit 2% Wirkstoff

| | |
|---|---|
| Wirkstoff des Beispiels Nr. 3 1-(4-Chlorphenyl)-1-(2,4-dichlorphenyl)-2-(1,2,4-triazol-1-yl)-äthan-1-ol | 2,0 g |
| Zinkoxid | 10,0 g |
| Magnesiumoxid | 10,0 g |
| Hochdisperses Siliciumdioxid | 2,5 g |
| Magnesiumstearat | 1,0 g |
| Talkum | 74,5 g |

Herstellung:

Der Wirkstoff wird auf einer Luftstrahlmühle mikronisiert und mit den anderen Bestandteilen homogen vermischt. Die Mischung wird durch Sieb Nr. 7 geschlagen und in Polyäthylenbehälter mit Streueinsatz abgefüllt.

## Patentansprüche

1. 1,1-Diphenyl-2-(1,2,4-triazol-1-yl)-äthanole der allgemeinen Formel I

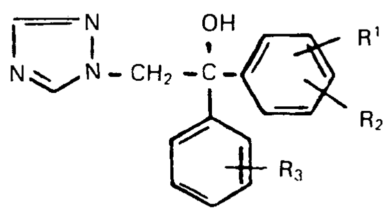

(I)

in der einer der Reste $R^1$ bis $R^3$ ein Chloratom bedeutet und die anderen, die gleich oder verschieden voneinander sein können, ein Wasserstoffatom oder Chloratom bedeuten, sowie deren pharmakologisch verträglichen Salze, als Antimykotika.

2. 1,1-Bis-(4-chlorphenyl)-2-(1,2,4-triazol-1-yl)-äthan-1-ol sowie seine pharmakologisch verträglichen Salze als Antimykotikum.

3. 1-Phenyl-1-(2,4-dichlorphenyl)-2-(1,2,4-tri-:azol-1-yl)-äthan-1-ol sowie seine pharmakologisch verträglichen Salze als Antimykotikum.

4. 1-(4-Chlorphenyl)-1-(2,4-dichlorphenyl)-2-(1,2,4-triazol-1-yl)-äthan-1-ol sowie seine pharmakologisch verträglichen Salze als Antimykotikum.

5. Antimykotikum, gekennzeichnet durch einen Gehalt an einer Verbindung gemäss einem der Ansprüche 1 bis 4 als Wirkstoff neben üblichen Hilfs- und Trägerstoffen.

## Revendications

1. Diphényl-1,1 (triazol-1,2,4 yl-1)-2 éthanols de la formule générale I

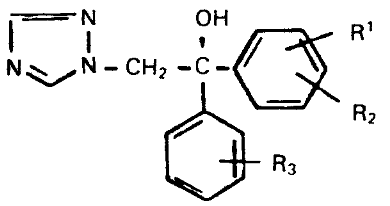

(I)

dans laquelle l'un des substituants $R^1$ à $R^3$ désigne un atome de chlore et les deux autres, qui peuvent être identiques ou différents, désignent chacun un atome d'hydrogène ou un atome de chlore, ainsi que leurs sels physiologiquement compatibles, comme agents antimycotiques.

2. Le bis-(chloro-4 phényl)-1,1 (triazol-1,2,4 yl-1)-2 éthanol-1 et ses sels physiologiquement compatibles comme agents antimycotiques.

3. Le phényl-1 (dichloro-2,4 phényl)-1 (triazol-1,2,4 yl-1)-2 éthanol-1 et ses sels physiologiquement compatibles comme agents antimycotiques.

4. Le (chloro-4 phényl)-1 (dichloro-2,4 phényl)-1 (triazol-1,2,4 yl-1)-2 éthanol-1 et ses sels physiologiquement compatibles comme agents antimycotiques.

5. Agent antimycotique, caractérisé en ce qu'il contient comme principe actif un composé selon l'une des revendications 1 à 4 à côtés d'autres additifs et véhicules usuels.

**Claims**

1. A 1,1-diphenyl-2-(1,2,4-triazol-1-yl)-ethanol of the general formula I

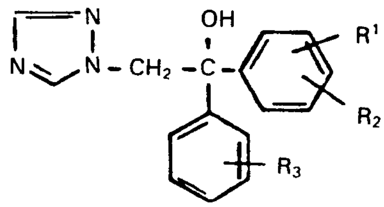

(I)

where one of the radicals $R^1$, $R^2$ and $R^3$ is chlorine and the others, which may be identical or different, are hydrogen or chlorine, and its pharmacologically tolerated salts as antimycotics.

2. 1,1-Bis-(4-chlorophenyl)-2-(1,2,4-triazol-1-yl)-ethan-1-ol and its pharmacologically tolerated salts as antimycotics.

3. 1-Phenyl-1-(2,4-dichlorophenyl)-2-(1,2,4-triazol-1-yl)-ethan-1-ol and its pharmacologically tolerated salts as antimycotics.

4. 1-(4-Chlorophenyl)-1-(2,4-dichlorophenyl)-2-(1,2,4-triazol-1-yl)-ethan-1-ol and its pharmacologically tolerated salts as antimycotics.

5. An antimycotic which contains a compound as claimed in any of claims 1 to 4 as the active compound, in addition to conventional auxiliaries and carriers.

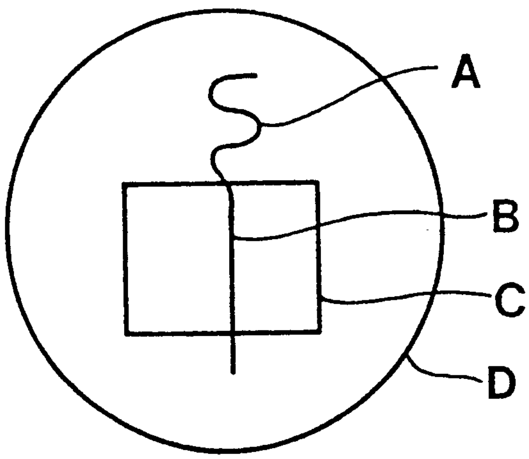
A
B
C
D